# FASCICULE DE BREVET EUROPEEN

(11) **EP 0 537 053 B1**
(45) Date de publication et mention de la délivrance du brevet: **28.01.1998**
(21) Numéro de dépôt: 92402690.9
(22) Date de dépôt: 02.10.1992
(51) Int. Cl.: C08F 220/04, A61L 15/00

(54) **Nouveaux polymères absorbants, procédé de fabrication et leur application notamment aux articles d'hygiène**
Absorbierende Polymere, Verfahren zu deren Herstellung und deren Verwendung in hygienen Artikeln
Absorbant polymers, process for their preparation and their use in sanitary articles

(30) Priorité: 11.10.1991 FR 9112552
(43) Date de publication de la demande: 14.04.1993
(73) Titulaire: Clariant GmbH, 65929 Frankfurt am Main (DE)
(72) Inventeur: Mallo, Paul, F-78400 Chatou (FR); Engelhardt, Fritz, W-6000 Frankfurt am Main 80 (DE); Riegel, Ulrich, W-6000 Frankfurt am Main 80 (DE); Funk, Rüdiger, W-6200 Wiesbaden Naurod (DE)

(56) Documents cités:
- EP-A- 0 457 660
- FR-A- 2 614 027
- GB-A- 2 126 591
- US-A- 4 735 987

## Description

La présente demande concerne des polymères absorbants, un procédé pour y accéder et l'application desdits polymères comme agent absorbant.

Des polymères réticulés, hydrophiles, insolubles dans l'eau à base d'acide acrylique et d'acrylate de métal alcalin contenant éventuellement des charges minérales telles que la silice sont connus et ils sont largement utilisé aujourd'hui dans des articles d'hygiène en raison de leur étonnant pouvoir absorbant des fluides physiologiques: urine, sang, etc ... . Le marché en expansion pour ce type de produit, est toujours demandeur soit de produits plus performants, soit plus économiques, soit plus écologiques. Notamment, on recherche des absorbants ne libérant pas des fluides absorbés lorsqu'ils sont soumis à des contraintes de pression. Or, la demanderesse a découvert avec étonnement des polymères absorbants, réticulés, hydrophiles, insoluble dans l'eau, sous forme de microperles, à base d'acide acrylique partiellement salifié par un métal alcalin. Ces polymères très absorbants présentent des propriétés absorbantes supérieures aux produits actuellement commercialisés ou décrits. Notamment, les polymères sont doués d'une très bonne capacité d'absorption d'eau sous charge.

C'est pourquoi la présente demande a pour objet les polymères absorbants, réticulés, hydrophiles, renfermant, à la température ambiante, moins de 5% de produits solubles dans l'eau, sous forme de microperles sensiblement sphériques de diamètre compris entre 0,05 et 1 mm, à base de silice colloidale, amorphe, à l'état de particules discrètes, d'un diamètre moyen compris entre 7 et 150 nm, non agglomérées entre elles par les liaisons siloxane : Si-O-Si et d'acide acrylique partiellement salifié par le sodium ou le potassium caractérisés par le fait qu'ils peuvent être obtenus par un procédé de polymérisation en suspension eau dans huile constituée par un ou plusieurs hydrocarbures non miscibles à l'eau, inertes vis-à-vis des amorceurs de polymérisation et susceptibles de fournir avec l'eau un azéotrope, en atmosphère inerte, dans lequel on introduit lentement, sous agitation, dans une phase huile parfaitement désoxygénée, maintenue à l'ébullition et contenant un colloide protecteur, une phase aqueuse obtenue extemporanément à partir d'une part, d'une solution aqueuse contenant un ou plusieurs amorceurs de polymérisation hydrosolubles, générateurs de radicaux libres, et d'autre part, une phase aqueuse contenant à une concentration de 50 ± 15 % en poids un mélange contenant pondéralement de 2 à 25 % de silice colloidale et de 98 à 75 % d'acide acrylique dont 60 à 80 % sont salifiés par le sodium ou le potassium, avec un rapport pondéral phase aqueuse sur phase huile compris entre 0,8 et 1,2, puis lorsque la réaction de polymérisation est terminée, l'on élimine par distillation azéotropique avec recyclage du solvant organique de 15 à 55 % de l'eau présente, l'on introduit ensuite dans le milieu réactionnel à l'ébullition de 0,01 à 0,06 % en proportions molaires par rapport aux monomères d'éthylèneglycol diglycidyl éther et l'on poursuit la distillation azéotropique de l'eau résiduelle jusqu'à l'obtention d'une suspension présentant un taux de siccité de 85 ± 10 % et l'on isole enfin le polymère cherché.

Actuellement, on ne sait pas exactement comment la silice colloidale est fixée dans les polymères de la présente invention. On sait cependant que les particules discrètes de silice sont uniformément réparties dans les microperles des polymères.

Parmi les polymères selon la présente invention, on peut citer plus particulièrement les polymères acide acrylique-acrylate de métal alcalin-silice ci-dessus pour lesquels le métal alcalin est le potassium.

On peut citer également ceux caractérisés par le fait que la réticulation est réalisée avec de 0,015 à 0,045 % en proportions molaires par rapport aux monomères d'éthylèneglycol diglycidyl éther ainsi que ceux caractérisés par le fait que la phase huile est le cyclohexane.

La présente invention a aussi pour objet un procédé de préparation de polymères absorbants, réticulés, hydrophiles, renfermant, à la température ambiante, moins de 5% de produits solubles dans l'eau, sous forme de microperles sensiblement sphériques de diamètre compris entre 0,05 et 1 mm, à base de silice colloidale, amorphe, à l'état de particules discrètes, d'un diamètre moyen compris entre 7 et 150 nm, non agglomérées entre elles par les liaisons siloxane : Si-O-Si et d'acide acrylique partiellement salifié par le sodium ou le potassium caractérisé en ce que l'on effectue une réaction de polymérisation en suspension eau dans huile constituée par un ou plusieurs hydrocarbures non miscibles à l'eau, inertes vis-à-vis des amorceurs de polymérisation et susceptibles de fournir avec l'eau un azéotrope, en atmosphère inerte, en introduisant lentement, sous agitation, dans une phase huile parfaitement désoxygénée, maintenue à l'ébullition, et contenant un colloide protecteur, une phase aqueuse obtenue extemporanément (de préférence au fur et à mesure de son introduction), à partir d'une part, d'une solution aqueuse contenant un ou plusieurs amorceurs de polymérisation hydrosolubles, générateurs de radicaux libres, et d'autre part, d'une phase aqueuse contenant à une concentration de 50 ± 15 % en poids un mélange contenant pondéralement de 2 à 25 % de silice colloidale et de 98 à 75 % d'acide acrylique dont 60 à 80 % sont salifié par le sodium ou le potassium, avec un rapport pondéral phase aqueuse sur phase huile compris entre 0,8 et 1,2, puis lorsque la réaction de polymérisation est terminée, que l'on élimine par distillation azéotropique partiellement l'eau, de préférence 15 à 55 % de celle-ci, avec recyclage du solvant organique entrainé, que l'on introduit ensuite dans le milieu réactionnel maintenu à l'ébullition de l'éthylèneglycol diglycidyl éther dans une proportion de 0,01 à 0,06 % en proportions molaires par rapport aux monomères engagés, que l'on poursuit la distillation azéotropique de l'eau résiduelle jusqu'à obtention d'une suspension présentant un taux de siccité de 85 ± 10 % en poids et qu'enfin l'on isole le polymère cherché, notamment par filtration.

La phase huile est constituée par un ou plusieurs hydrocarbures non miscibles à l'eau, inertes vis-à-vis des amorceurs de polymérisation et susceptibles de fournir avec l'eau un azéotrope. On peut citer par exemple des fractions d'essence de point d'ébullition compris entre 50 et 180°C et notamment le cyclohexane.

Le colloïde protecteur, de préférence utilisé à la dose de 0,4 à 2 % en poids par rapport au poids des monomères, est choisi parmi ceux couramment utilisés dans ce type de polymérisation en suspension (cf. Kirk-Othmer, Encyclopedia of Chemical Technology, 3ème édition, volume 1, page 400). Avantageusement, on choisira un éther de cellulose et, préférentiellement, un éthyléther de cellulose présentant un taux d'éthoxyle de 48 à 49,5 % (cf. Encyclopedia of Polymer Science and Engineering, 2ème édition, volume 3, page 254). Le colloïde protecteur est de préférence préalablement dissous ou dispersé dans la phase huile.

La réaction de polymérisation est effectuée à l'ébullition du milieu réactionnel, habituellement à la pression ambiante. Elle peut également être effectuée à une pression inférieure ou supérieure à la pression ambiante. La réaction de polymérisation est amorcée par un ou plusieurs générateurs de radicaux libres, hydrosolubles; ceux-ci présentent avantageusement une demi-vie à 70 °C supérieure à deux heures. De tels agent d'amorçage sont notamment certains péroxydes minéraux comme le péroxodisulfate de sodium ou certains azoïques comme l'acide dicyano-4,4' azopentanedioïque-4,4'. ILs sont avantageusement utilisés en solution aqueuse à la concentration de 200 à 3000 ppm par rapport au poids des monomères et préférentiellement à une concentration de 500 à 1500 ppm. L'acrylate de métal alcalin est obtenu avantageusement en solution aqueuse, par salification directe d'une solution aqueuse d'acide acrylique avec l'hydroxyde de métal alcalin correspondant, de préférence l'hydroxyde de potassium .

Cette salification est avantageusement effectuée à une température comprise entre 20° et 35°C. Les monomères mis en oeuvre sont utilisés en solution aqueuse.

La silice est apportée sous forme de suspensions aqueuses concentrées de particules non agglomérées de silice amorphe telles que celles commercialisées par la demanderesse sous la désignation "KLEBOSOL".

La solution aqueuse d'amorçage et la phase aqueuse contenant les monomères et la silice à une concentration pondérale de 50 ± 15 % sont mélangées extemporanément, préférentiellement au fur et à mesure de leur introduction lente dans la phase huile agitée, parfaitement désoxygénée et maintenue à l'ébullition par un chauffage extérieur si nécessaire. Le rapport pondéral phase aqueuse sur phase huile est compris entre 0,8 et 1,2, de préférence entre 0,95 et 1,05 et avantageusement ce rapport est environ égal à 1.

La durée d'introduction peut varier selon les unités opératoires, mais généralement elle est comprise entre une et deux heures. En fin d'introduction, il est avantageux de maintenir le milieu réactionnel à l'ébullition pendant 10 à 60 minutes, sous agitation, pour parfaire la polymérisation. La polymérisation terminée, on élimine partiellement, de préférence de 15 à 55 %, l'eau présente par distillation azéotropique avec recyclage du solvant organique ainsi entraîné , puis on introduit dans le milieu réactionnel maintenu à l'ébullition, de 100 à 600 ppm, avantageusement de 150 à 450 ppm en proportions molaires par rapport aux monomères, d'éthylèneglycol diglycidyl éther, et ensuite, on poursuit l'élimination de l'eau présente dans le milieu réactionnel par distillation azéotropique jusqu'à obtention d'une suspension présentant un taux de siccité de 85 ± 10 % et l'on isole le produit attendu. En vue de cette isolation, la suspension peut être filtrée et le polymère obtenu selon le procédé de l'invention ainsi recueilli, est séché jusqu'à un taux de siccité supérieur à 90 %. On obtient ainsi un polymère réticulé insoluble dans l'eau, sous forme de microperles exemptes de fines, à fort pouvoir hydrophile et à très faible taux de monomères résiduels, lesquels sont toujours inférieurs à 0,01 % en poids.

Les propriétés hydrophiles des polymères selon l'invention sont déterminées aisément par un ensemble de tests simples.

Ainsi, la capacité d'absorption d'eau du polymère, désignée TG, est déterminée à 20°C, en agitant durant 30 minutes, 0,4 g de polymère dans 500 g d'eau, puis en pesant le gel polymère obtenu égoutté. Le poids trouvé est ramené à 1 g de polymère sec. Les copolymères de la présente invention présentent dans ce test une capacité d'absorption de l'ordre de 300 à 700 g par gramme de polymère sec. Par "polymère sec", l'on désigne un polymère à 100 % de matières actives.

La capacité d'absorption de solution physiologique salée du polymère, désignée TGS, est déterminée à 20°C, en agitant durant 30 minutes, 2 g de polymère dans 500 g d'une solution physiologique salée puis en pesant le gel polymère obtenu égoutté. Le poids trouvé est ramené à 1 g de polymère sec. Les copolymères de la présente invention présentent dans ce test une capacité d'absorption de l'ordre de 40 à 70 g par gramme de polymère sec.

Le taux d'extractibles, désigné TE, est déterminé selon la méthode suivante :
- on place 1 g de polymère à tester dans 200 g de solution physiologique salée ;
- on agite cette suspension une heure à 20°C, puis on l'abandonne 15 heures au repos à 20°C ;
- on égoutte le gel polymère obtenu et on recueille le filtrat ;
- on dose sur 100 cm³ du filtrat les fonctions carboxyliques et carboxylates présentes ;
- on exprime le résultat de ce dosage en gramme de polymère dissous pour 100 g de polymère sec.

Dans ce test, les polymères de la présente invention présentent un taux d'extractibles de 1 à 5 %.

La capacité d'absorption par capillarité sous une charge de 15 g par cm², désignée TGC, est déterminée à 20°C selon le protocole suivant : dans un entonnoir cylindrique à plaque filtrante d'un diamètre de 90 mm et de porosité 1, on étale successivement et uniformément, 40 g de sable de Fontainebleau de granulométrie 0,100 à 0,300 mm, 2 g de copolymère à tester et enfin 40 g de sable de Fontainebleau. On place ensuite sur la couche supérieure de sable par l'intermédiaire d'un disque de verre de diamètre 90 mm, une charge totale de 954 g, puis l'entonnoir est plongé dans un bac contenant une solution physiologique salée, à niveau constant, de manière que le niveau de l'eau affleure rigoureusement la face supérieure du fritté et on mesure la quantité de solution physiologique salée absorbée par capillarité par le copolymère en 90 minutes. On exprime le résultat en g de solution physiologique salée par gramme de polymère sec.

Les polymères selon la présente invention présentent d'intéressantes propriétés absorbantes qui justifient leur application comme agent absorbant et l'invention a également pour objet, à titre d'agents absorbants, les polymères tels que définis ci-dessus, notamment pour la fabrication d'articles d'hygiène particulièrement de couches pour bébés.

Les exemples suivants illustrent la présente invention sans toutefois la limiter.

### EXEMPLE 1

On disperse en atmosphère inerte, dans 635 g de cyclohexane :
- 3,5 g d'éthyléther de cellulose, désigné EEC, contenant de 48 à 49,5 % de groupements éthoxylés et présentant à 25°C, une viscosité de 200 mPa.s en solution à 5 % dans un mélange toluène-éthanol 80-20 en poids. On obtient ainsi une dispersion désignée D.

Par ailleurs, on dissout sous agitation, à une température inférieure à 30°C, 230 g (3,19 moles) d'acide acrylique, désigné AA, dans 300 g d'une solution aqueuse d'hydroxyde de potassium contenant 171 g d'eau et 129 g (2,3 moles) d'hydroxyde de potassium, puis dans cette solution on introduit successivement 80 g d'un sol de silice contenant 30 % en poids de silice sous forme de particules non liées entre elles, d'un diamètre moyen de 13 nm, présentant un pH de 9 et stabilisé avec 0,3 % en poids d'hydroxyde de potassium, 317 mg de péroxodisulfate de sodium dissous dans 10 g d'eau et enfin 13,7 g d'eau de manière à amener la masse totale de la phase aqueuse à 634 g.

La phase aqueuse préparée ci-dessus est ensuite introduite sous agitation, en atmosphère inerte, en 90 minutes dans la dispersion D soigneusement désoxygénée et maintenue à l'ébullition par un chauffage extérieur.

Au fur et à mesure de l'introduction de la phase aqueuse dans la dispersion D, on observe une réaction exothermique.

A la fin de l'introduction, on maintient le milieu réactionnel une heure à l'ébullition, puis on le soumet à une distillation azéotropique avec recyclage du cyclohexane de manière à éliminer environ 50 g d'eau.

A ce stade, à l'ébullition et sous agitation, on introduit dans le milieu réactionnel 111,4 mg (0,639 mmole) d'éthylèneglycol diglycidyl éther, puis on poursuit la distillation azéotropique comme précédemment jusqu'à l'élimination de 200 g environ d'eau.

La suspension obtenue est alors refroidie à la température ambiante, puis elle est filtrée et le précipité recueilli est séché dans une étuve ventilée jusqu'à ce qu'il présente un taux de siccité de 96 % environ.

On obtient ainsi 355 g (341 g exprimé en sec) d'un polymère acide acrylique-acrylate de potassium-silice, insoluble dans l'eau, et se présentant sous forme de perles de quelques dixièmes de millimètres de diamètre. Ce polymère présente une capacité d'absorption d'eau, désignée TG, d'environ 310 g/g, et une capacité d'absorption de solution physiologique salée , désignée TGS, d'environ 46 g/g et une capacité d'absorption de solution physiologique salée sous charge, désignée TGC, d'environ 27 g/g. Le taux d'extractibles est de 0,7 %.

### EXEMPLES 2-7

On reproduit l'exemple 1 en modifiant d'une part, la quantité d'eau éliminée par distillation azéotropique avant l'introduction de l'éthylèneglycol diglycidyl éther, désignée PE, et d'autre part, la quantité d'éthylèneglycol diglycidyl éther utilisée, désignée PG. Le tableau I mentionne ces diverses quantités ainsi que les propriétés d'absorption des différents polymères obtenus.

**TABLEAU I**

| **EXEMPLE** | **PE** | **PG** | **TG** | **TGS** | **TGC** |
|---|---|---|---|---|---|
| 1 | 50 g | 111,4 mg | 310 g/g | 46 g/g | 27 g/g |
| 2 | 130 g | 111,4 mg | 464 g/g | 52 g/g | 24,5 g/g |
| 3 | 181 g | 111,4 mg | 569 g/g | 55 g/g | 24 g/g |
| 4 | 100 g | 167 mg | 326 g/g | 45,5 g/g | 28 g/g |
| 5 | 130 g | 167 mg | 400 g/g | 49,5 g/g | 28,5 g/g |
| 6 | 151 g | 167 mg | 444 g/g | 52 g/g | 27 g/g |
| 7 | 130 g | 167 mg | 401 g/g | 49 g/g | 29 g/g |

## Revendications

1. Polymères absorbants, réticulés, hydrophiles, renfermant, à la température ambiante, moins de 5% de produits solubles dans l'eau, sous forme de microperles sensiblement sphériques de diamètre compris entre 0,05 et 1 mm, à base de silice colloidale, amorphe, à l'état de particules discrètes, d'un diamètre moyen compris entre 7 et 150 nm, non agglomérées entre elles par les liaisons siloxane : Si-O-Si et d'acide acrylique partiellement salifié par le sodium ou le potassium caractérisés par le fait qu'ils peuvent être obtenus par un procédé de polymérisation en suspension eau dans huile constituée par un ou plusieurs hydrocarbures non miscibles à l'eau, inertes vis-à-vis des amorceurs de polymérisation et susceptibles de fournir avec l'eau un azéotrope, en atmosphère inerte, dans lequel on introduit lentement, sous agitation, dans une phase huile parfaitement désoxygénée, maintenue à l'ébullition et contenant un colloide protecteur, une phase aqueuse obtenue extemporanément à partir d'une part, d'une solution aqueuse contenant un ou plusieurs amorceurs de polymérisation hydrosolubles, générateurs de radicaux libres, et d'autre part, une phase aqueuse contenant à une concentration de 50 ± 15 % en poids un mélange contenant pondéralement de 2 à 25 % de silice colloidale et de 98 à 75 % d'acide acrylique dont 60 à 80 % sont salifiés par le sodium ou le potassium, avec un rapport pondéral phase aqueuse sur phase huile compris entre 0,8 et 1,2, puis lorsque la réaction de polymérisation est terminée, l'on élimine par distillation azéotropique avec recyclage du solvant organique de 15 à 55 % de l'eau présente, l'on introduit ensuite dans le milieu réactionnel à l'ébullition de 0,01 à 0,06 % en proportions molaires par rapport aux monomères d'éthylèneglycol diglycidyl éther et l'on poursuit la distillation azéotropique de l'eau résiduelle jusqu'à l'obtention d'une suspension présentant un taux de siccité de 85 ± 10 % et l'on isole enfin le polymère cherché.

2. Polymères selon la revendication 1 caractérisés par le fait que l'acide acrylique est salifié par le potassium.

3. Polymères selon l'une quelconque des revendications 1 ou 2 caractérisées par le fait que la réticulation est réalisée avec de 0,015 à 0,045 % en proportions molaires par rapport aux monomères d'éthylèneglycol diglycidyl éther.

4. Polymères selon l'une quelconque des revendications 1 à 3 caractérisés par le fait que la phase huile est le cyclohexane.

5. Procédé de préparation de polymères absorbants, réticulés, hydrophiles, renfermant, à la température ambiante, moins de 5% de produits solubles dans l'eau, sous forme de microperles sensiblement sphériques de diamètre compris entre 0,05 et 1 mm, à base de silice colloidale, amorphe, à l'état de particules discrètes, d'un diamètre moyen compris entre 7 et 150 nm, non agglomérées entre elles par les liaisons siloxane : Si-O-Si et d'acide acrylique partiellement salifié par le sodium ou le potassium caractérisé en ce que l'on effectue une réaction de polymérisation en suspension eau dans huile constituée par un ou plusieurs hydrocarbures non miscibles à l'eau, inertes vis-à-vis des amorceurs de polymérisation et susceptibles de fournir avec l'eau un azéotrope, en atmosphère inerte, en introduisant lentement, sous agitation, dans une phase huile parfaitement désoxygénée, maintenue à l'ébullition, et contenant un colloide protecteur, une phase aqueuse obtenue extemporanément, à partir d'une part, d'une solution aqueuse contenant un ou plusieurs amorceurs de polymérisation hydrosolubles, générateurs de radicaux libres, et d'autre part, d'une phase aqueuse contenant à une concentration de 50 ± 15 % en poids un mélange contenant pondéralement de 2 à 25 % de silice colloidale et de 98 à 75 % d'acide acrylique dont 60 à 80 % sont salifiés par le sodium ou le potassium, avec un rapport pondéral phase aqueuse sur phase huile compris entre 0,8 et 1,2, puis lorsque la réaction de polymérisation est terminée, que l'on élimine par distillation azéotropique partiellement l'eau, de préférence 15 à 55 % de celle-ci, avec recyclage du solvant organique entrainé, que l'on introduit ensuite dans le milieu réactionnel maintenu à l'ébullition de l'éthylèneglycol diglycidyl éther dans une proportion de 0,01 à 0,06 % en proportions molaires par rapport aux monomères engagés, que l'on poursuit la distillation azéotropique de l'eau résiduelle jusqu'à obtention d'une suspension présentant un taux de siccité de 85 + 10 % en poids et qu'enfin l'on isole le polymère cherché.

6. Procédé selon la revendication 5, caractérisé par le fait que l'acide acrylique est salifié par le potassium.

7. Procédé selon la revendication 5 ou 6, caractérisé par le fait que la réticulation est réalisée avec de 0,015 à 0,045 % en proportions molaires par rapport aux monomères d'éthylèneglycol diglycidyl éther.

8. Procédé selon la revendication 5, 6 ou 7, caractérisé par le fait que la phase huile est le cyclohexane.

9. Application d'un polymère hydrophile selon l'une quelconque des revendications 1 à 4 comme composé absorbant.

10. Application selon la revendication 9 dans un article d'hygiène.

## Claims

1. Hydrophilic, crosslinked, absorbent polymers containing, at ambient temperature, less than 5 % of water-soluble products, in the form of substantially spherical microbeads between 0.05 and 1 mm in diameter, based on amorphous colloidal silica in the state of discrete particles of a mean diameter between 7 and 150 nm, not agglomerated together by siloxane bonds: Si-O-Si and on acrylic acid partially converted into the sodium or potassium salt, characterized in that they can be obtained by a polymerization process in water suspension in oil consisting of one or several water-immiscible hydrocarbons which are inert towards polymerization initiators and capable of forming an azeotrope with water, in an inert atmosphere, in which an aqueous phase obtained extemporaneously from, on the one hand, an aqueous solution containing one or several water-soluble polymerization initiators which generate free radicals and, on the other hand, an aqueous phase containing in a concentration of 50 ± 15 % by weight a mixture containing, by weight, from 2 to 25 % of colloidal silica and from 98 to 75 % of acrylic acid of which 60 to 80 % is converted into the sodium or potassium salt is introduced slowly, with stirring, into a perfectly deoxygenated oil phase kept boiling and containing a protective colloid, with a weight ratio of aqueous phase to oil phase of between 0.8 and 1.2, then when the polymerization reaction is finished 15 to 55 % of the water present is removed by azeotropic distillation with recycling of the organic solvent, next from 0.01 to 0.06 % of ethylene glycol diglycidyl ether in molar proportions relative to the monomers is introduced into the boiling reaction mixture and the azeotropic distillation of the residual water is continued until a suspension is obtained exhibiting a degree of dryness of 85 ± 10 %, and the required polymer is finally isolated.

2. Polymers according to claim 1, characterized in that the acrylic acid is converted into salt with potassium.

3. Polymers according to either of claims 1 and 2, characterized in that the crosslinking is carried out with 0.015 to 0.45 % of ethylene glycol diglycidyl ether in molar proportions relative to the monomers.

4. Polymers according to any one of claims 1 to 3, characterized in that the oil phase is cyclohexane.

5. Process for preparation of hydrophilic, crosslinked, absorbent polymers containing, at ambient temperature, less than 5 % of water-soluble products, in the form of substantially spherical microbeads between 0.05 and 1 mm in diameter, based on amorphous colloidal silica in the state of discrete particles of a mean diameter between 7 and 150 nm, not agglomerated together by siloxane bonds: Si-O-Si and on acrylic acid partially converted into the sodium or potassium salt, characterized in that a polymerization reaction is performed in water suspension in oil consisting of one or several water-immiscible hydrocarbons which are inert towards polymerization initiators and capable of forming an azeotrope with water, in an inert atmosphere, an aqueous phase obtained extemporaneously from, on the one hand, an aqueous solution containing one or several water-soluble polymerization initiators which generate free radicals and, on the other hand, an aqueous phase containing in a concentration of 50 ± 15 % by weight a mixture containing, by weight from 2 to 25 % of colloidal silica and from 98 to 75 % of acrylic acid of which 60 to 80 % is converted into the sodium or potassium salt being introduced slowly, with stirring, into a perfectly deoxygenated oil phase kept boiling and containing a protective colloid, with a weight ratio of aqueous phase to oil phase of between 0.8 and 1.2, then when the polymerization reaction is finished, the water, preferably from 15 to 55 % of the latter, is partially removed by azeotropic distillation with recycling of the entrained organic solvent, in that next from 0.01 to 0.06 % of ethylene glycol diglycidyl ether in molar proportions relative to the monomers involved is introduced into the reaction mixture which is kept boiling and in that the azeotropic distillation of the residual water is continued until a suspension is obtained exhibiting a degree of dryness of 85 ± 10 % by weight, and in that the required polymer is finally isolated.

6. Process according to claim 5, characterized in that the acrylic acid is converted into salt with potassium.

7. Process according to claim 5 or 6, characterized in that crosslinking is carried out with from 0.015 to 0.045 % of ethylene glycol diglycidyl ether in molar proportions relative to the monomers.

8. Process according to claim 5, 6 or 7, characterized in that the oil phase is cyclohexane.

9. Application of a hydrophobic polymer according to any one of claims 1 to 4 as an absorbent compound.

10. Application according to claim 9 in an article of hygiene.

## Patentansprüche

1. Absorbierende, vernetzte, hydrophile, bei Umgebungstemperatur weniger als 5% wasserlösliche Produkte enthaltende, in Form von im wesentlichen kugelförmigen Mikroperlen mit einem Durchmesser zwischen 0,05 und 1 mm vorliegende Polymere auf der Basis von amorpher, kolloidaler Kieselsäure in Form von einzelnen Teilchen mit einem mittleren Durchmesser zwischen 7 und 150 nm, die untereinander durch Si-O-Si-Siloxanbindungen nicht agglomeriert sind, und von mittels Natrium oder Kalium teilweise in die Salzform überführter Acrylsäure, dadurch gekennzeichnet, daß sie nach einem Polymerisationsverfahren in einer Wasser-in-Öl-Suspension, die aus einem oder mehreren mit Wasser nicht mischbaren, gegenüber Polymerisationsinitiatoren reaktionsträgen und zur Bildung eines Azeotrops mit Wasser befähigten Kohlenwasserstoffen besteht, unter einer Schutzgasatmosphäre erhältlich sind, wobei man in die vollständig von Sauerstoff befreite Ölphase, die am Sieden gehalten wird und ein Schutzkolloid enthält, unter Rühren langsam eine wäßrige Phase einträgt, die einerseits aus einer wäßrigen Lösung, die einen oder mehrere wasserlösliche, freie Radikale bildende Polymerisationsinitiatoren enthält, und andererseits aus einer wäßrigen Phase, die eine Mischung aus 2 bis 25 Gew.-% kolloidaler Kieselsäure und 98 bis 75 Gew.-% Acrylsäure, die zu 60 bis 80% mit Natrium oder Kalium in die Salzform überführt worden ist, in einer Konzentration von 50 ± 15 Gew.-% enthält, mit einem Gewichtsverhältnis der wäßrigen Phase zur Ölphase zwischen 0,8 und 1,2 in-situ hergestellt wurde, und anschließend nach Beendigung der Polymerisationsreaktion unter Rückgewinnung des organischen Lösungsmittels 15 bis 55% des vorhandenen Wassers azeotrop abdestilliert, anschließend in das am Sieden gehaltene Reaktionsmedium 0,01 bis 0,06 Mol% Ethylenglykoldiglycidylether, bezogen auf die Monomeren, einträgt und das restliche Wasser solange azeotrop weiter abdestilliert, bis man eine Suspension mit einem Trockengehalt von 85 ± 10% erhält, und das gesuchte Polymer schließlich isoliert.

2. Polymere nach Anspruch 1, dadurch gekennzeichnet, daß die Acrylsäure durch Kalium in die Salzform überführt wird.

3. Polymere nach einem der Ansprüche 1 oder 2, dadurch gekennzeichnet, daß die Vernetzung mittels 0,015 bis 0,045 Mol% Ethylenglykol-diglycidylether, bezogen auf die Monomere, erfolgt.

4. Polymere nach einem der Ansprüche 1 bis 3, dadurch gekennzeichnet, daß die Ölphase aus Cyclohexan besteht.

5. Verfahren zur Herstellung von absorbierenden, vernetzten, hydrophilen, bei Umgebungstemperatur weniger als 5% wasserlösliche Produkte enthaltenden, in Form von im wesentlichen kugelförmigen Mikroperlen mit einem Durchmesser zwischen 0,05 und 1 mm vorliegenden Polymeren auf der Basis amorpher, kolloidaler Kieselsäure in Form von einzelnen Teilchen mit einem mittleren Durchmesser zwischen 7 und 150 nm, die untereinander durch Si-O-Si-Siloxanbindungen nicht agglomeriert sind, und von mittels Natrium oder Kalium teilweise in die Salzform überführter Acrylsäure, dadurch gekennzeichnet, daß man eine Polymerisationsreaktion in einer Wasser-in-Öl-Suspension, die aus einem oder mehreren mit Wasser nicht mischbaren, gegenüber Polymerisationsinitiatoren reaktionsträgen und zur Bildung eines Azeotrops mit Wasser befähigten Kohlenwasserstoffen besteht, unter einer Schutzgasatmosphäre durchführt, indem man in eine vollständig von Sauerstoff befreite Ölphase, die am Sieden gehalten wird und ein Schutzkolloid enthält, unter Rühren langsam eine wäßrige Phase einträgt, die einerseits aus einer wäßrigen Lösung, die einen oder mehrere wasserlösliche, freie Radikale bildende Polymerisationsinitiatoren enthält, und andererseits aus einer wäßrigen Phase, die eine Mischung aus 2 bis 25 Gew.-% kolloidaler Kieselsäure und 98 bis 75 Gew.-% Acrylsäure, die zu 60 bis 80% mit Natrium oder Kalium in die Salzform überführt worden ist, in einer Konzentration von 50 ± 15 Gew.-% enthält, mit einem Gewichtsverhältnis der wäßrigen Phase zur Ölphase zwischen 0,8 und 1,2 in-situ hergestellt wurde, und anschließend nach Beendigung der Polymerisationsreaktion unter Rückgewinnung des mitgeführten organischen Lösungsmittels das Wassers teilweise, vorzugsweise zu 15 bis 55%, azeotrop abdestilliert, anschließend in das am Sieden gehaltene Reaktionsmedium Ethylenglykoldiglycidylether in einem Anteil von 0,01 bis 0,06 Mol%, bezogen auf die beteiligten Monomere, einträgt, das restliche Wasser solange azeotrop weiter abdestilliert, bis man eine Suspension mit einem Trockengehalt von 85 ± 10 Gew.-% erhält, und das gesuchte Polymer schließlich isoliert.

6. Verfahren nach Anspruch 5, dadurch gekennzeichnet, daß die Acrylsäure mit Kalium in die Salzform überführt wird.

7. Verfahren nach Anspruch 5 oder 6, dadurch gekennzeichnet, daß die Vernetzung mittels 0,015 bis 0,045 Mol% Ethylenglykol-diglycidylether, bezogen auf die Monomere, erfolgt.

8. Verfahren nach Anspruch 5, 6 oder 7, dadurch gekennzeichnet, daß die Ölphase aus Cyclohexan besteht.

9. Verwendung von hydrophilen Polymeren nach einem der Ansprüche 1 bis 4 als absorbierende Komponente.

10. Verwendung nach Anspruch 9 in Hygieneartikeln.
